# EUROPEAN PATENT APPLICATION

(11) **EP 4 696 699 A1**
(43) Date of publication of application: **18.02.2026**
(21) Application number: 24787969.5
(22) Date of filing: 03.04.2024
(51) Int. Cl.: C07K 7/06, A61K 38/08

(54) **? OPIOID RECEPTOR AGONIST AND USE THEREOF**

(30) Priority: 10.04.2023 CN 202310371594; 26.01.2024 CN 202410112984
(71) Applicant: Chengdu Aoda Biotechnology Co., Ltd., Chengdu, Sichuan 610041 (CN)
(72) Inventor: ZHOU, Shuliang, Chengdu, Sichuan 610041 (CN); WANG, Peng, Chengdu, Sichuan 610041 (CN); DENG, Lan, Chengdu, Sichuan 610041 (CN)
(74) Representative: PGA S.p.A., Milano, Succursale di Lugano
(86) International application number: PCT/CN2024/085849
(87) International publication number: WO 2024/212865

(57) **Abstract**

A κ opioid receptor agonist and a stereoisomer and pharmaceutically acceptable salt thereof, having the following structural formula and relating to the technical field of biological medicines. The κ opioid receptor agonist has high selectivity to a κ opioid receptor, the activity of the κ opioid receptor agonist is 3-15 times that of Difelikefalin, the drug activity is high, and the κ opioid receptor agonist can be used for treating and/or relieving pruritus and/or analgesia. D-Phe-AA1-AA2-D-Lys-4-aminopiperidine-4-carboxamide-AA3-AA4-AA5 (Formula I).

## Description

This application claims the priority of Chinese Patent Application No. 202310371594.6, filed with the China National Intellectual Property Administration on April 10, 2023, and titled "LONG-ACTING K-OPIOID RECEPTOR AGONIST", and Chinese Patent Application No. 202410112984.6, filed with the China National Intellectual Property Administration on January 26, 2024, and titled "LONG-ACTING K-OPIOID RECEPTOR AGONIST", which are hereby incorporated by reference in their entirety.

### FIELD

The present disclosure relates to the field of biomedical technology, and in particular to a high-selectivity κ-opioid receptor agonist and use thereof.

### BACKGROUND

Opioid drugs exert physiological effects mainly through binding to three known classical opioid receptors µ, δ, and κ. These three receptors are all members of the G-protein-coupled receptor family, are mainly distributed in the central nervous system, and also exist in many peripheral tissues.

The most classical one of these drugs is morphine, which exerts an analgesic effect mainly through the µ-opioid receptor. Clinically common analgesic drugs also include other µ-opioid receptor drugs, such as traditional opioid drugs as represented by dihydromorphinone and fentanyl. However, long-term use of µ-opioid receptor drugs can lead to multiple side effects, such as tolerance, dependence, respiratory depression, effects on gastrointestinal motility, and the like, which not only increase treatment costs, but also affect recovery periods of patients. Some non-opioid injections, such as acetaminophen and non-steroidal anti-inflammatory drugs, have limited application ranges and dosages due to their poor analgesic effects; furthermore, they also have certain side effects, for example, acetaminophen increases liver toxicity, and non-steroidal anti-inflammatory drugs can cause various gastrointestinal diseases.

κ-opioid receptor exists in brain, spinal cord, central and peripheral nerve endings, somatic and visceral sensory afferent nerve cell bodies, and immune cells. Research has found that, using κ-opioid receptor agonists, the κ-opioid receptor can be used as a therapeutic target for treating pain and preventing a wide variety of diseases and conditions, for example, the treatment of pain including hyperalgesia, the use in eye disorders and eye pain, the treatment of pruritus caused by uremia and opioids, and the like.

Difelikefalin (brand name KORSUVA) is a first-in-class high-selectivity κ-opioid receptor (KOR) full agonist, which can inhibit activity of pruritus-producing peripheral neurons, and has no significant activity on other receptors (including µ- or δ- opioid receptors), ion channels, or transporters. Besides, unlike small-molecule KOR agonists, Korsuva is a small synthetic peptide, which mainly activates KORs expressed by peripheral neurons (PNS) and immune cells. The U.S. FDA has approved Difelikefalin for marketing for the treatment of moderate to severe pruritus related to chronic kidney disease in adults (CKD-aP) undergoing hemodialysis (HD). Difelikefalin has exhibited antipruritic and analgesic properties, as well as inhibition of acute postoperative pain (IV preparations in laparoscopic hysterectomy, and resection of ventral hernia and bunion), in several different studies of Phase II or III.

The present disclosure is intended to provide a highly active κ-opioid receptor agonist.

### SUMMARY

An object of the present disclosure is to provide a class of peptide compounds that have high selectivity for κ-opioid receptor, and are novel, highly active κ-opioid receptor agonists. A further object of the present disclosure is to provide a pharmaceutical composition. Another object of the present disclosure is to provide a use of the compounds and the pharmaceutical composition.

To achieve the above objects, the present disclosure provides the following technical solutions:

In a first aspect, the present disclosure provides a κ-opioid receptor agonist having a structure as represented by formula I, a stereisomer thereof, and a pharmaceutically acceptable salt thereof:

D-Phe-AA1-AA2-D-Lys-4-aminopiperidine-4-carboxamide-AA3-AA4-AA5 (formula I)

wherein, AA1 is modified or unmodified D-Phe, or modified or unmodified D-Tyr;
AA2 is D-Leu, or D-cyclopropylalanine;
AA3 is -(PEGₘ₁(CH₂)ₘ₂CO)ₘ₃-, or (AA6)ₘ₄, or is absent, wherein:
m₁ is an integer ranging from 1 to 10;
m₂ is an integer ranging from 1 to 5;
m₃ is an integer ranging from 1 to 5;
m₄ is an integer ranging from 1 to 5;
AA6 is AEEA, or Gly, or D-Ala, or L-Ala, or D-Leu, or L-Leu, or D-Phe, or L-Phe, or D-Ser, or L-Ser, or D-Thr, or L-Thr, or D-Tyr, or L-Tyr, or D-Asp, or L-Asp, or D-Glu, or L-Glu, or D-Gln, or L-Gln, or D-Lys, or L-Lys, or D-Arg, or L-Arg, or D-His, or L-His;
AA4 is (AA7)ₙ, or is absent, wherein:
n is an integer ranging from 1 to 10;
AA7 is D-Lys, or L-Lys, or D-Dap, or L-Dap, or D-Dab, or L-Dab, or D-Orn, or L-Orn, or D-Dah, or L-Dah, or D-Dao, or L-Dao;
AA5 is OH, or NH₂.

Preferably, AA1 is modified D-Phe, and the modified D-Phe is selected from the group consisting of D-Phe(2-F), D-Phe(4-F), D-Phe(2-Cl), D-Phe(4-Cl), D-Phe(2-Br), D-Phe(4-Br), D-Phe(2-I), D-Phe(4-I), D-Phe(4-Me), D-Phe(4-Et), D-Phe(4-Ipr), D-Phe(4-NH₂), D-Phe(4-NHCH₃), D-Phe(4-NHCH₂CH₃), D-Phe(4-NHCH(CH₃)₂), D-Phe(4-N(CH₃)₂), and D-Phe(4-N(CH₃) CH₂CH₃).

Preferably, AA1 is modified D-Tyr, and the modified D-Tyr is selected from the group consisting of D-Tyr(Me), D-Tyr(Et), D-Tyr(CH₂CF₃) and D-Tyr(Ipr).

Further, the κ-opioid receptor agonist is a compound having a structure as represented by formula II, a stereisomer thereof, and a pharmaceutically acceptable salt thereof:

D-Phe-AA1-AA2-D-Lys-4-aminopiperidine-4-carboxamide-R-(AA8)ₚ-NH₂(OH) (formula II)

Unless otherwise specified, in structural formulas of the present disclosure, "-NH₂(OH)" represents -NH₂ or -OH.

Wherein, AA1 is modified D-Phe, or modified D-Tyr, wherein the specific modifications are as described above;
AA2 is D-Leu, or D-cyclopropylalanine;
R is present or absent, and when R is present, R is selected from the group consisting of-PEGₘ₁CH₂CO-,-AEEAₙ₁-, and-Glyₙ₂-; wherein:
m₁ is an integer ranging from 1 to 5, and specifically, is selected from the group consisting of 1, 2, 3, 4, and 5;
n₁ is an integer ranging from 1 to 5, and specifically, is selected from the group consisting of 1, 2, 3, 4, and 5;
n₂ is an integer ranging from 1 to 5, and specifically, is selected from the group consisting of 1, 2, 3, 4, and 5;
AA8 is Lys, or D-Lys; p is an integer ranging from 0 to 6, and specifically, is selected from the group consisting of 0, 1, 2, 3, 4, 5, and 6; when p is 0, AA8 is absent.

In an optional embodiment of the present disclosure, the κ-opioid receptor agonist is a compound having a structure as represented by formula III, a stereisomer thereof, and a pharmaceutically acceptable salt thereof:

D-Phe-D-Tyr(X)-D-Leu-D-Lys-4-aminopiperidine-4-carboxamide-R₁-(AA8)ₚt-NH₂(OH) (formula III)

wherein, D-tyr(X) represents modified D-Tyr; the modified D-Tyr is selected from the group consisting of D-Tyr(Me), D-Tyr(Et), D-Tyr(CH₂CF₃), and D-Tyr(Ipr);
R₁ is present or absent; when R₁ is present, R₁ is selected from the group consisting of-PEGₖ₁CH₂CO-, and-Glyₙ₂-; wherein:
   k₁ is an integer ranging from 1 to 5, and specifically, is selected from the group consisting of 1, 2, 3, 4, and 5;
   n₂ is an integer ranging from 1 to 5, and specifically, is selected from the group consisting of 1, 2, 3, 4, and 5;
   AA8 is Lys, or D-Lys; p₁ is an integer ranging from 0 to 6, and specifically, is selected from the group consisting of 0, 1, 2, 3, 4, 5, and 6; when p₂ is 0, AA8 is absent.

In an optional embodiment of the present disclosure, the κ-opioid receptor agonist is a compound having a structure as represented by formula IV, a stereisomer thereof, and a pharmaceutically acceptable salt thereof:

D-Phe-D-Tyr(X)-D-cyclopropylalanine-D-Lys-4-aminopiperidine-4-carboxamide -R₂-(AA8)ₚ₂-NH₂(OH) (formula IV)

wherein, D-tyr(X) represents modified D-Tyr; the modified D-Tyr is selected from the group consisting of D-Tyr(Me), D-Tyr(Et), and D-Tyr(Ipr).

R₂ is present or is absent; when R₂ is present, R₂ is-PEGₖ₂CH₂CO-; wherein:
k₂ is an integer ranging from 1 to 5, and specifically, is selected from the group consisting of 1, 2, 3, 4, and 5;
in most preferred embodiments of the present disclosure, the k₂ is 1, 2, or 3;
AA8 is Lys, or D-Lys; p₃ is an integer ranging from 0 to 6, and specifically, is selected from the group consisting of 0, 1, 2, 3, 4, 5, and 6; when p₃ is 0, AA8 is absent;
in most preferred embodiments of the present disclosure, the p₃ is 0 or 1.

In an optional embodiment of the present disclosure, the κ-opioid receptor agonist is a compound having a structure as represented by formula V, a stereisomer thereof, and a pharmaceutically acceptable salt thereof:

D-Phe-D-Phe(X)-D-Leu-D-Lys-4-aminopiperidine-4-carboxamide-(AA8)ₚ₃-NH₂(OH) (formula V)

wherein, D-Phe(X) represents modified D-Phe; the modified D-Phe is selected from the group consisting of D-Phe(2-F), D-Phe(4-F), D-Phe(2-Cl), D-Phe(4-Cl), D-Phe(2-Br), D-Phe(4-Br), D-Phe(2-I), D-Phe(4-I), D-Phe(4-Me), D-Phe(4-Et), D-Phe(4-Ipr), D-Phe(4-NH₂).

AA8 is Lys, or D-Lys; p₃ is an integer ranging from 0 to 6, and specifically, is selected from the group consisting of 0, 1, 2, 3, 4, 5, and 6; when p₃ is 0, AA8 is absent;
in most preferred embodiments of the present disclosure, the p₃ is 0 or 1.
further preferably, the κ-opioid receptor agonist has one or more structure formulas selected from the group consisting of 1) to 10):
   1) D-Phe-D-Tyr(X)-D-Leu-D-Lys-4-aminopiperidine-4-carboxylic acid-NH₂(OH)
   2) D-Phe-D-Tyr(X)-D-Leu-D-Lys-4-aminopiperidine-4-carboxamide-NH₂(OH)
   3) D-Phe-D-Tyr(X)-D-Leu-D-Lys-4-aminopiperidine-4-carboxamide-(AA8)_{q}-NH₂(OH)
   4) D-Phe-D-Tyr(X)-D-Leu-D-Lys-4-aminopiperidine-4-carboxamide-PEGₖ₁CH₂CO-AA8-NH₂(OH)
   5) D-Phe-D-Tyr(X)-D-Leu-D-Lys-4-aminopiperidine-4-carboxamide-Glyₙ₂-AA8-NH₂(OH)
   6) D-Phe-D-Tyr(X)-D-cyclopropylalanine-D-Lys-4-aminopiperidine-4-carboxamide-NH₂(OH)
   7) D-Phe-D-Tyr(X)-D-cyclopropylalanine-D-Lys-4-aminopiperidine-4-carboxamide-AA8-NH₂(OH)
   8) D-Phe-D-Tyr(X)-D-cyclopropylalanine-D-Lys-4-aminopiperidine-4-carboxamide-PEGₖ₂CH₂CO-AA8-NH₂(OH)
   9) D-Phe-D-Phe(X)-D-Leu-D-Lys-4-aminopiperidine-4-carboxamide-NH₂(OH)
   10) D-Phe-D-Phe(X)-D-Leu-D-Lys-4-aminopiperidine-4-carboxamide-AA8-NH₂(OH),
in aforementioned structural formulas, AA8 is Lys, or D-Lys; wherein:
   in formula 1), D-Tyr(X) is D-Tyr(Et);
   in formulas 2) to 3), D-Tyr(X) is D-Tyr(Me), or D-Tyr(Et), or D-Tyr(CH₂CF₃), or D-Tyr(Ipr), and q is 1, or 2, or 3, or 4, or 5, or 6;
   in formulas 4) to 8), D-Tyr(X) is D-Tyr(Et),
   k₁ is 1, or 2, or 3, or 4, or 5;
   n₂ is 1, or 2, or 3, or 4, or 5;
   k₂ is 1, or 2, or 3;
   in formula 9), D-Phe(X) is D-Phe(4-F), or D-Phe(4-Cl), or D-Phe(4-Br), or D-Phe(4-I);
   in formula 10), D-Phe(X) is D-Phe(4-F), or D-Phe(4-Cl), or D-Phe(4-Br), or D-Phe(4-I), or D-Phe(4-Me), or D-Phe(4-Et), or D-Phe(4-Ipr), or D-Phe(4-NH₂);

In a second aspect, the present disclosure provides a pharmaceutical composition, wherein the pharmaceutical composition comprises the κ-opioid receptor agonist provided in the present disclosure, the stereisomer thereof, and the pharmaceutically acceptable salt thereof.

Further, the pharmaceutical composition further comprises a pharmaceutically acceptable carrier.

In a third aspect, the present disclosure provides use of the κ-opioid receptor agonist, the stereisomer, and the pharmaceutically acceptable salt thereof, and/or the pharmaceutical composition, in the manufacture of a medicament for preventing and/or relieving pruritus and/or an analgesic.

The κ-opioid receptor agonist provided in the present disclosure has a high selectivity for κ-opioid receptor, with an activity 3 to 15 times that of the marketed drug Difelikefalin. The high drug activity makes it useful for treating, preventing and/or relieving pruritus and/or for combating pain.

### DETAILED DESCRIPTION

As used in the present disclosure, the term "stereoisomer" refers to compounds that have the same chemical constitution but differ in the spatial arrangement of their atoms or groups. Stereoisomers include, but are not limited to, enantiomers, diastereomers, conformational isomers (rotamers), geometric (cis/trans) isomers, and atropisomers.

The "pharmaceutical composition" described in the present disclosure can refer to a composition for the treatment of a disease, or for use in in vitro cell culture experiments. When intended for the treatment of a disease, the term "pharmaceutical composition" typically refers to a unit dosage form and can be prepared by any of the methods well known in the field of pharmacy. In general, all such methods include the step of bringing the active ingredient into association with an excipient which constitutes one or more accessory components.

As used herein, the term "pharmaceutically acceptable" refers to a substance or composition that is chemically and/or toxicologically compatible with the other ingredients of a formulation and/or the mammal being treated therewith. Preferably, "pharmaceutically acceptable" refers to what is approved by a regulatory agency of a federal or national government or listed in the U.S. Pharmacopeia or another generally recognized pharmacopeia for use in animals, and more particularly in humans.

Specifically, the term "pharmaceutically acceptable carrier" can include any and all solvents, solid excipients, diluents, liquid vehicles, and the like, suitable for the particular dosage form desired. The use of any conventional carriers is also contemplated by the present disclosure, except insofar as they are incompatible with the κ-opioid receptor agonist provided by the present disclosure, for example, by producing any undesirable biological effect or otherwise interacting in a deleterious manner with any other component of the pharmaceutically acceptable composition.

The technical solutions in the embodiments of the present disclosure will be described clearly and completely below. It should be understood that the described embodiments are merely some, but not all, of the embodiments of the present disclosure. Based on the embodiments in the present disclosure, all other embodiments obtained by a person of ordinary skill in the art without exercising creative effort shall fall within the scope of protection of the present disclosure.

As used herein, the terms "alleviate", "ameliorate", or "mitigate" may be used interchangeably. These terms refer to a method of obtaining a beneficial or desired result, including but not limited to, a therapeutic benefit. "Therapeutic benefit" means the eradication or amelioration of the underlying disorder being treated. As used herein, a therapeutic benefit is achieved by the eradication or amelioration of one or more physiological symptoms associated with the underlying disorder, such that an improvement is observed in the subject, although the subject may still be afflicted with the underlying disorder.

The present disclosure provides a κ-opioid receptor agonist and uses thereof. A person skilled in the art can, by referring to the content of this disclosure, suitably modify the relevant parameters to practice the present disclosure. While the methods of the present disclosure have been described by preferred examples, it will be apparent to those skilled in the art that modifications, or suitable variations and combinations, can be made to the compounds and preparation methods described herein without departing from the content, spirit, and scope of the present disclosure to realize and apply the technology of the present disclosure.

The full names corresponding to the English abbreviations used in the present disclosure are shown in the table below:

**Table 1**

| **English Abbreviations** | **Full Name** | **English Abbreviations** | **Full Name** |
|---|---|---|---|
| Fmoc | 9-fluorenylmethylox ycarbonyl | OtBu | tert-butoxy |
| tBu | tert-butyl | Boc | tert-butoxycarbonyl |
| Trt | trityl | Pbf | (2,3-dihydro-2,2,4,6,7-pentameth ylbenzofuran-5-yl)sulfonyl |
| Ala | alanine | Lys | lysine |
| Arg | arginine | Phe | phenylalanine |
| Asn | asparagine | Ser | serine |
| Gln | glutamine | Thr | threonine |
| Glu | glutamic acid | Trp | tryptophan |
| Gly | glycine | Tyr | tyrosine |
| His | histidine | Val | valine |
| Leu | leucine | Dap | 2,3-diaminopropionic acid |
| 5-Ava | 5-aminovaleric acid | Dab | 2,4-diaminobutyric acid |
| Ada | 2-aminoadipic acid | Orn | ornithine |
| Apm | 2-aminopimelic acid | Dah | 2,7-diaminoheptanoic acid |
| Asu | 2-aminosuberic acid | Dao | 2,8-diaminooctanoic acid |

### Example 1: preparation of compounds

The preparation method is solid-phase polypeptide synthesis, which comprises: preparing a peptide-resin by solid-phase polypeptide synthesis, cleaving the peptide-resin with an acid to obtain a crude product, and finally purifying the crude product to obtain a pure product. Wherein, the step of preparing the peptide-resin by solid-phase polypeptide synthesis involves sequentially coupling the corresponding protected amino acids of the following sequence onto a carrier resin via solid-phase coupling synthesis method to prepare the peptide-resin:

in the aforementioned preparation method, the Fmoc-protected amino acid is used at an amount of 1.2 to 6 times the total molar amount of the resin used; preferably 2.5 to 3.5 times.

In the aforementioned preparation method, the carrier resin has a substitution value of 0.3 to 1.5 mmol/g resin, preferably 0.6 to 1.0 mmol/g resin.

In a preferred embodiment of the disclosure, the solid-phase coupling synthesis method is as follows: the Fmoc protecting group is removed from the protected amino acid-resin obtained from the previous step, followed by a coupling reaction with the next protected amino acid. The deprotection step of removing the Fmoc group had a duration of 10 to 60 min, preferably 15 to 25 min. The coupling reaction had a duration of 60 to 300 min, preferably 100 to 140 min.

The coupling reaction requires the addition of a condensation reagent. The condensation reagent is selected from the group consisting of DIC (N,N-diisopropylcarbodiimide), N,N-dicyclohexylcarbodiimide, benzotriazol-1-yl-oxy-tripyrrolidinophosphonium hexafluorophosphate, 2-(7-aza-1H-benzotriazol-1-yl)-1,1,3,3-tetramethyluronium hexafluorophosphate, benzotriazol-N,N,N',N'-tetramethyluronium hexafluorophosphate, and O-(benzotriazol)-N,N,N',N'-tetramethyluronium tetrafluoroborate. The preferred one is N,N-diisopropylcarbodiimide. The condensation reagent is used at a molar amount of 1.2 to 6 times the total molar amount of amino groups on the amino-resin, preferably 2.5 to 3.5 times.

The coupling reaction requires the addition of an activating agent. The activating agent is selected from the group consisting of 1-hydroxybenzotriazole and N-hydroxy-7-azabenzotriazole, preferably 1-hydroxybenzotriazole. The activating agent is used at an amount of 1.2 to 6 times the total molar amount of amino groups on the amino-resin, preferably 2.5 to 3.5 times.

In a preferred embodiment of the present disclosure, the reagent for removing the Fmoc protecting group is a mixed solution of PIP/DMF (piperidine/N,N-dimethylformamide), wherein the mixed solution contains 10% to 30% (V) piperidine. The amount of the Fmoc deprotection reagent used is 5 to 15 mL per gram of amino-resin, preferably 8 to 12 mL per gram of amino-resin.

More preferably, acidolysis agent used in the step of acidolysis of the peptide-resin is a mixed solvent of trifluoroacetic acid (TFA), 1,2-ethanedithiol (EDT), and water, wherein the mixed solvent has a volume ratio of: 80% to 95% TFA, 1% to 10% EDT, with the balance being water.

Even more preferably, the mixed solvent has a volume ratio of: 89% to 91% TFA, 4% to 6% EDT, with the balance being water. Most preferably, the mixed solvent has a volume ratio of: 90% TFA, 5% EDT, with the balance being water.

The acidolysis agent is used at an amount of 4 to 15 mL per gram of peptide-resin; preferably, 7 to 10 mL per gram of peptide-resin.

The cleavage step using the acidolysis agent has a duration of 1 to 6 h, preferably 3 to 4 h, at room temperature.

Furthermore, the crude product is purified by high-performance liquid chromatography and then lyophilized to obtain the pure product.

### 1. Synthesis of peptide-resin

Peptide-resin was prepared by taking a carrier resin and sequentially coupling the corresponding protected amino acids of the sequence via deprotection of Fmoc and coupling reactions:

### (1) Coupling of the first protected amino acid of the main chain

0.03 mol of the first protected amino acid and 0.03 mol of HOBt were dissolved in an appropriate amount of DMF. Separately, 0.03 mol of DIC was added slowly to the DMF solution of the protected amino acid with stirring. Reaction was performed at room temperature under stirring for 30 min to obtain a solution of activated protected amino acid, which was set aside for later use.

0.01 mol of carrier resin (substitution value was approximately 0.4 mmol/g) was taken, and deprotected by treatment with a 20% PIP/DMF solution for 25 min. The Fmoc-deprotected resin was obtained after washing and filtering.

The activated first protected amino acid solution was added to the Fmoc-deprotected resin, and the coupling reaction was carried out for 60 to 300 min. The resin containing one protected amino acid was obtained after filtering and washing.

### (2) Coupling of other protected amino acids

Using the same method as for the coupling of the first amino acid of the sequence, the other corresponding protected amino acids of the sequence were sequentially coupled to obtain a resin containing the peptides.

### 2. Preparation of the crude product

The peptide-resin obtained above was treated with a cleavage reagent (10 mL of cleavage reagent per gram of resin) with a volume ratio of TFA:water:EDT = 95:5:5. The mixture was stirred evenly and allowed to react at room temperature for 3 h. The reaction mixture was filtered through a fritted funnel, and the filtrate was collected. The resin was washed three times with a small amount of TFA. The filtrates were combined and concentrated under reduced pressure. Anhydrous diethyl ether was added to precipitate the peptide. Then the precipitate was washed three times with anhydrous diethyl ether and then dried under vacuum to yield an off-white powder.

### 3. Preparation of the pure product

The crude product concentrate obtained above was purified by filtering through a 0.45 µm mixed microporous filter membrane and set aside for later use.

Purification was performed using high-performance liquid chromatography. The chromatographic packing material for purification was 10 µm reverse-phase C18 material, and the mobile phase system consisted of 0.1% TFA/water and 0.1% TFA/acetonitrile. For a 30 mm*250 mm chromatography column, the flow rate was 20 mL/min. A gradient elution and cyclic loading purification was used. The crude product solution was loaded onto the column, and the mobile phase was initiated for elution. The main peak was collected, and after removing acetonitrile by evaporation, a purified intermediate concentrate was obtained.

The purified intermediate concentrate was filtered through a 0.45 µm membrane and set aside for later use. Salt exchange was performed using high-performance liquid chromatography. The mobile phase system was 1% acetic acid/water-acetonitrile, and the chromatographic packing material for purification was 10 µm reverse-phase C18 material. For a 30 mm*250 mm chromatography column, the flow rate was 20 mL/min (the flow rate can be adjusted according to different chromatography column specifications). A gradient elution and cyclic loading method was used. The sample was loaded onto the column, the mobile phase was initiated for elution, and chromatograms were collected. Changes in absorbance were observed, and the main peak from the salt exchange was collected. The purity was checked by analytical liquid chromatography. The fractions containing the main peak from the salt exchange were combined, concentrated under reduced pressure to obtain a purified product aqueous solution in acetic acid, and then lyophilized to obtain the pure product.

Using the method described above, the following compounds were synthesized:

**Table 2**

| **Compound Number** | **Sequence** |
|---|---|
| Difelikefalin | D-Phe-D-Phe-D-Leu-D-Lys-4-aminopiperidine-4-carboxylic acid-OH |
| Compound 1 | D-Phe-D-Tyr(Et)-D-Leu-D-Lys-4-aminopiperidine-4-carboxylic acid-NH₂ |
| Compound 2 | D-Phe-D-Tyr(Et)-D-Leu-D-Lys-4-aminopiperidine-4-carboxylic acid-OH |
| Compound 3 | D-Phe-D-Tyr(Et)-D-Leu-D-Lys-4-aminopiperidine-4-carboxamide-L ys-NH₂ |
| Compound 4 | D-Phe-D-Tyr(Et)-D-Leu-D-Lys-4-aminopiperidine-4-carboxamide-L ys-OH |
| Compound 5 | D-Phe-D-Tyr(Et)-D-Leu-D-Lys-4-aminopiperidine-4-carboxamide-L ys-Lys-NH₂ |
| Compound 6 | D-Phe-D-Tyr(Et)-D-Leu-D-Lys-4-aminopiperidine-4-carboxamide-L ys-Lys-OH |
| Compound 7 | D-Phe-D-Tyr(Et)-D-Leu-D-Lys-4-aminopiperidine-4-carboxamide-L ys-Lys-Lys-NH₂ |
| Compound 8 | D-Phe-D-Tyr(Et)-D-Leu-D-Lys-4-aminopiperidine-4-carboxamide-L ys-Lys-Lys-OH |
| Compound 9 | D-Phe-D-Tyr(Et)-D-Leu-D-Lys-4-aminopiperidine-4-carboxamide-L ys-Lys-Lys- Lys-NH₂ |
| Compound 10 | D-Phe-D-Tyr(Et)-D-Leu-D-Lys-4-aminopiperidine-4-carboxamide-L ys-Lys-Lys- Lys-OH |
| Compound 11 | D-Phe-D-Tyr(Et)-D-Leu-D-Lys-4-aminopiperidine-4-carboxamide-L ys-Lys-Lys- Lys- Lys- NH₂ |
| Compound 12 | D-Phe-D-Tyr(Et)-D-Leu-D-Lys-4-aminopiperidine-4-carboxamide-L ys-Lys-Lys- Lys- Lys- OH |
| Compound 13 | D-Phe-D-Tyr(Et)-D-Leu-D-Lys-4-aminopiperidine-4-carboxamide-L ys-Lys-Lys-Lys-Lys-Lys-NH₂ |
| Compound 14 | D-Phe-D-Tyr(Et)-D-Leu-D-Lys-4-aminopiperidine-4-carboxamide-L ys-Lys-Lys-Lys-Lys-Lys-OH |
| Compound 15 | D-Phe-D-Tyr(Et)-D-Leu-D-Lys-4-aminopiperidine-4-carboxamide-D -Lys-NH₂ |
| Compound 16 | D-Phe-D-Tyr(Et)-D-Leu-D-Lys-4-aminopiperidine-4-carboxamide-D -Lys-OH |
| Compound 17 | D-Phe-D-Tyr(Et)-D-Leu-D-Lys-4-aminopiperidine-4-carboxamide-D -Lys-D-Lys-NH₂ |
| Compound 18 | D-Phe-D-Tyr(Et)-D-Leu-D-Lys-4-aminopiperidine-4-carboxamide-D -Lys-D-Lys-OH |
| Compound 19 | D-Phe-D-Tyr(Et)-D-Leu-D-Lys-4-aminopiperidine-4-carboxamide-D -Lys-D-Lys-D-Lys-NH₂ |
| Compound 20 | D-Phe-D-Tyr(Et)-D-Leu-D-Lys-4-aminopiperidine-4-carboxamide-D -Lys-D-Lys-D-Lys-OH |
| Compound 21 | D-Phe-D-Tyr(Et)-D-Leu-D-Lys-4-aminopiperidine-4-carboxamide-D -Lys-D-Lys-D-Lys-D-Lys -NH₂ |
| Compound 22 | D-Phe-D-Tyr(Et)-D-Leu-D-Lys-4-aminopiperidine-4-carboxamide-D -Lys-D-Lys-D-Lys-D-Lys -OH |
| Compound 23 | D-Phe-D-Tyr(Et)-D-Leu-D-Lys-4-aminopiperidine-4-carboxamide-D -Lys-D-Lys-D-Lys-D-Lys -D-Lys-NH₂ |
| Compound 24 | D-Phe-D-Tyr(Et)-D-Leu-D-Lys-4-aminopiperidine-4-carboxamide-D -Lys-D-Lys-D-Lys-D-Lys -D-Lys-OH |
| Compound 25 | D-Phe-D-Tyr(Et)-D-Leu-D-Lys-4-aminopiperidine-4-carboxamide-D -Lys-D-Lys-D-Lys-D-Lys -D-Lys-D-Lys-NH₂ |
| Compound 26 | D-Phe-D-Tyr(Et)-D-Leu-D-Lys-4-aminopiperidine-4-carboxamide-D -Lys-D-Lys-D-Lys-D-Lys -D-Lys-D-Lys-OH |
| Compound 27 | D-Phe-D-Tyr(Et)-D-Leu-D-Lys-4-aminopiperidine-4-carboxamide-P EG₁CH₂CO-Lys-NH₂ |
| Compound 28 | D-Phe-D-Tyr(Et)-D-Leu-D-Lys-4-aminopiperidine-4-carboxamide-P EG¡CH₂CO-Lys-OH |
| Compound 29 | D-Phe-D-Tyr(Et)-D-Leu-D-Lys-4-aminopiperidine-4-carboxamide-P EGiCH₂CO-D-Lys-NH₂ |
| Compound 30 | D-Phe-D-Tyr(Et)-D-Leu-D-Lys-4-aminopiperidine-4-carboxamide-P EG₁CH₂CO-D-Lys-OH |
| Compound 31 | D-Phe-D-Tyr(Et)-D-Leu-D-Lys-4-aminopiperidine-4-carboxamide-PEG₂CH₂CO -Lys-NH2 |
| Compound 32 | D-Phe-D-Tyr(Et)-D-Leu-D-Lys-4-aminopiperidine-4-carboxamide-PEG₂CH₂CO -Lys-OH |
| Compound 33 | D-Phe-D-Tyr(Et)-D-Leu-D-Lys-4-aminopiperidine-4-carboxamide-PEG₂CH₂CO -D-Lys-NH₂ |
| Compound 34 | D-Phe-D-Tyr(Et)-D-Leu-D-Lys-4-aminopiperidine-4-carboxamide-PEG₂CH₂CO -D-Lys-OH |
| Compound 35 | D-Phe-D-Tyr(Et)-D-Leu-D-Lys-4-aminopiperidine-4-carboxamide-P EG₃CH₂CO-Lys-NH₂ |
| Compound 36 | D-Phe-D-Tyr(Et)-D-Leu-D-Lys-4-aminopiperidine-4-carboxamide-P EG₃CH₂CO-Lys-OH |
| Compound 37 | D-Phe-D-Tyr(Et)-D-Leu-D-Lys-4-aminopiperidine-4-carboxamide-P EG₃CH₂CO-D-Lys-NH₂ |
| Compound 38 | D-Phe-D-Tyr(Et)-D-Leu-D-Lys-4-aminopiperidine-4-carboxamide-P EG₃CH₂CO-D-Lys-OH |
| Compound 39 | D-Phe-D-Tyr(Et)-D-Leu-D-Lys-4-aminopiperidine-4-carboxamide-P EG₄CH₂CO-Lys-NH₂ |
| Compound 40 | D-Phe-D-Tyr(Et)-D-Leu-D-Lys-4-aminopiperidine-4-carboxamide-P EG₄CH₂CO-Lys-OH |
| Compound 41 | D-Phe-D-Tyr(Et)-D-Leu-D-Lys-4-aminopiperidine-4-carboxamide-P EG₄CH₂CO-D-Lys-NH₂ |
| Compound 42 | D-Phe-D-Tyr(Et)-D-Leu-D-Lys-4-aminopiperidine-4-carboxamide-P EG₄CH₂CO-D-Lys-OH |
| Compound 43 | D-Phe-D-Tyr(Et)-D-Leu-D-Lys-4-aminopiperidine-4-carboxamide-P EG₅CH₂CO-Lys-NH₂ |
| Compound 44 | D-Phe-D-Tyr(Et)-D-Leu-D-Lys-4-aminopiperidine-4-carboxamide-P EG₅CH₂CO-Lys-OH |
| Compound 45 | D-Phe-D-Tyr(Et)-D-Leu-D-Lys-4-aminopiperidine-4-carboxamide-P EG₅CH₂CO-D-Lys-NH₂ |
| Compound 46 | D-Phe-D-Tyr(Et)-D-Leu-D-Lys-4-aminopiperidine-4-carboxamide-P EG₅CH₂CO-D-Lys-OH |
| Compound 47 | D-Phe-D-Tyr(Et)-D-Leu-D-Lys-4-aminopiperidine-4-carboxamide-G ly-Lys-NH₂ |
| Compound 48 | D-Phe-D-Tyr(Et)-D-Leu-D-Lys-4-aminopiperidine-4-carboxamide-G ly-Lys-OH |
| Compound 49 | D-Phe-D-Tyr(Et)-D-Leu-D-Lys-4-aminopiperidine-4-carboxamide-G ly-D-Lys-NH₂ |
| Compound 50 | D-Phe-D-Tyr(Et)-D-Leu-D-Lys-4-aminopiperidine-4-carboxamide-G ly-D-Lys-OH |
| Compound 51 | D-Phe-D-Tyr(Et)-D-Leu-D-Lys-4-aminopiperidine-4-carboxamide-G ly-Gly-Lys-NH₂ |
| Compound 52 | D-Phe-D-Tyr(Et)-D-Leu-D-Lys-4-aminopiperidine-4-carboxamide-G ly-Gly-Lys-OH |
| Compound 53 | D-Phe-D-Tyr(Et)-D-Leu-D-Lys-4-aminopiperidine-4-carboxamide-G ly-Gly-D-Lys-NH₂ |
| Compound 54 | D-Phe-D-Tyr(Et)-D-Leu-D-Lys-4-aminopiperidine-4-carboxamide-G ly-Gly-D-Lys-OH |
| Compound 55 | D-Phe-D-Tyr(Et)-D-Leu-D-Lys-4-aminopiperidine-4-carboxamide-G ly-Gly-Gly-Lys-NH₂ |
| Compound 56 | D-Phe-D-Tyr(Et)-D-Leu-D-Lys-4-aminopiperidine-4-carboxamide-G ly-Gly-Gly-Lys-OH |
| Compound 57 | D-Phe-D-Tyr(Et)-D-Leu-D-Lys-4-aminopiperidine-4-carboxamide-G ly-Gly-Gly-D-Lys-NH₂ |
| Compound 58 | D-Phe-D-Tyr(Et)-D-Leu-D-Lys-4-aminopiperidine-4-carboxamide-G ly-Gly-Gly-D-Lys-OH |
| Compound 59 | D-Phe-D-Tyr(Et)-D-Leu-D-Lys-4-aminopiperidine-4-carboxamide-G ly-Gly-Gly-Gly-Lys-NH₂ |
| Compound 60 | D-Phe-D-Tyr(Et)-D-Leu-D-Lys-4-aminopiperidine-4-carboxamide-G ly-Gly-Gly-Gly-Lys-OH |
| Compound 61 | D-Phe-D-Tyr(Et)-D-Leu-D-Lys-4-aminopiperidine-4-carboxamide-G ly-Gly-Gly-Gly-D-Lys-NH₂ |
| Compound 62 | D-Phe-D-Tyr(Et)-D-Leu-D-Lys-4-aminopiperidine-4-carboxamide-G ly-Gly-Gly-Gly-D-Lys-OH |
| Compound 63 | D-Phe-D-Tyr(Et)-D-Leu-D-Lys-4-aminopiperidine-4-carboxamide-G ly-Gly-Gly-Gly-Gly-Lys-NH₂ |
| Compound 64 | D-Phe-D-Tyr(Et)-D-Leu-D-Lys-4-aminopiperidine-4-carboxamide-G ly-Gly-Gly-Gly-Gly-Lys-OH |
| Compound 65 | D-Phe-D-Tyr(Et)-D-Leu-D-Lys-4-aminopiperidine-4-carboxamide-G ly-Gly-Gly-Gly-Gly-D-Lys-NH₂ |
| Compound 66 | D-Phe-D-Tyr(Et)-D-Leu-D-Lys-4-aminopiperidine-4-carboxamide-G ly-Gly-Gly-Gly-Gly-D-Lys-OH |
| Compound 67 | D-Phe-D-Tyr(Et)-D-cyclopropylalanine-D-Lys-4-aminopiperidine-4-carboxamide-NH₂ |
| Compound 68 | D-Phe-D-Tyr(Et)-D-cyclopropylalanine-D-Lys-4-aminopiperidine-4-carboxamide-OH |
| Compound 69 | D-Phe-D-Tyr(Et)-D-cyclopropylalanine-D-Lys-4-aminopiperidine-4-carboxamide-Lys-NH₂ |
| Compound 70 | D-Phe-D-Tyr(Et)-D-cyclopropylalanine-D-Lys-4-aminopiperidine-4-carboxamide-Lys-OH |
| Compound 71 | D-Phe-D-Tyr(Et)-D-cyclopropylalanine-D-Lys-4-aminopiperidine-4-carboxamide-D-Lys-NH₂ |
| Compound 72 | D-Phe-D-Tyr(Et)-D-cyclopropylalanine-D-Lys-4-aminopiperidine-4-carboxamide-D-Lys-OH |
| Compound 73 | D-Phe-D-Tyr(Et)-D-cyclopropylalanine-D-Lys-4-aminopiperidine-4-carboxamide- PEG₁CH₂CO-Lys-NH₂ |
| Compound 74 | D-Phe-D-Tyr(Et)-D-cyclopropylalanine-D-Lys-4-aminopiperidine-4-carboxamide- PEG₁CH₂CO-Lys-OH |
| Compound 75 | D-Phe-D-Tyr(Et)-D-cyclopropylalanine-D-Lys-4-aminopiperidine-4-carboxamide- PEG₁CH₂CO-D-Lys-NH₂ |
| Compound 76 | D-Phe-D-Tyr(Et)-D-cyclopropylalanine-D-Lys-4-aminopiperidine-4-carboxamide- PEG₁CH₂CO-D-Lys-OH |
| Compound 77 | D-Phe-D-Tyr(Et)-D-cyclopropylalanine-D-Lys-4-aminopiperidine-4-carboxamide- PEG₂CH₂CO-Lys-NH₂ |
| Compound 78 | D-Phe-D-Tyr(Et)-D-cyclopropylalanine-D-Lys-4-aminopiperidine-4-carboxamide- PEG₂CH₂CO-Lys-OH |
| Compound 79 | D-Phe-D-Tyr(Et)-D-cyclopropylalanine-D-Lys-4-aminopiperidine-4-carboxamide- PEG₂CH₂CO-D-Lys-NH₂ |
| Compound 80 | D-Phe-D-Tyr(Et)-D-cyclopropylalanine-D-Lys-4-aminopiperidine-4-carboxamide- PEG₂CH₂CO-D-Lys-OH |
| Compound 81 | D-Phe-D-Tyr(Et)-D-cyclopropylalanine-D-Lys-4-aminopiperidine-4-carboxamide- PEG₃CH₂CO-Lys-NH₂ |
| Compound 82 | D-Phe-D-Tyr(Et)-D-cyclopropylalanine-D-Lys-4-aminopiperidine-4-carboxamide- PEG₃CH₂CO-Lys-OH |
| Compound 83 | D-Phe-D-Tyr(Et)-D-cyclopropylalanine-D-Lys-4-aminopiperidine-4-carboxamide- PEG₃CH₂CO-D-Lys-NH₂ |
| Compound 84 | D-Phe-D-Tyr(Et)-D-cyclopropylalanine-D-Lys-4-aminopiperidine-4-carboxamide- PEG₃CH₂CO-D-Lys-OH |
| Compound 85 | D-Phe-D-Tyr(Me)-D-Leu-D-Lys-4-aminopiperidine-4-carboxamide-NH₂ |
| Compound 86 | D-Phe-D-Tyr(Me)-D-Leu-D-Lys-4-aminopiperidine-4-carboxamide-OH |
| Compound 87 | D-Phe-D-Tyr(Me)-D-Leu-D-Lys-4-aminopiperidine-4-carboxamide-Lys-NH₂ |
| Compound 88 | D-Phe-D-Tyr(Me)-D-Leu-D-Lys-4-aminopiperidine-4-carboxamide-Lys-OH |
| Compound 89 | D-Phe-D-Tyr(Me)-D-Leu-D-Lys-4-aminopiperidine-4-carboxamide-D-Lys-NH₂ |
| Compound 90 | D-Phe-D-Tyr(Me)-D-Leu-D-Lys-4-aminopiperidine-4-carboxamide-D-Lys-OH |
| Compound 91 | D-Phe-D-Tyr(CH₂CF₃)-D-Leu-D-Lys-4-aminopiperidine-4-carboxam ide-NH₂ |
| Compound 92 | D-Phe-D-Tyr(CH₂CF₃)-D-Leu-D-Lys-4-aminopiperidine-4-carboxam ide-OH |
| Compound 93 | D-Phe-D-Tyr(CH₂CF₃)-D-Leu-D-Lys-4-aminopiperidine-4-carboxam ide-Lys-NH₂ |
| Compound 94 | D-Phe-D-Tyr(CH₂CF₃)-D-Leu-D-Lys-4-aminopiperidine-4-carboxam ide-Lys-OH |
| Compound 95 | D-Phe-D-Tyr(CH₂CF₃)-D-Leu-D-Lys-4-aminopiperidine-4-carboxam ide-D-Lys-NH₂ |
| Compound 96 | D-Phe-D-Tyr(CH₂CF₃)-D-Leu-D-Lys-4-aminopiperidine-4-carboxam ide-D-Lys-OH |
| Compound 97 | D-Phe-D-Tyr(Ipr)-D-Leu-D-Lys-4-aminopiperidine-4-carboxamide-NH₂ |
| Compound 98 | D-Phe-D-Tyr(Ipr)-D-Leu-D-Lys-4-aminopiperidine-4-carboxamide-OH |
| Compound 99 | D-Phe-D-Tyr(Ipr)-D-Leu-D-Lys-4-aminopiperidine-4-carboxamide-L ys-NH₂ |
| Compound 100 | D-Phe-D-Tyr(Ipr)-D-Leu-D-Lys-4-aminopiperidine-4-carboxamide-L ys-OH |
| Compound 101 | D-Phe-D-Tyr(Ipr)-D-Leu-D-Lys-4-aminopiperidine-4-carboxamide-D-Lys-NH₂ |
| Compound 102 | D-Phe-D-Tyr(Ipr)-D-Leu-D-Lys-4-aminopiperidine-4-carboxamide-D-Lys-OH |
| Compound 103 | D-Phe-D-Phe(4-F)-D-Leu-D-Lys-4-aminopiperidine-4-carboxamide-NH₂ |
| Compound 104 | D-Phe-D-Phe(4-F)-D-Leu-D-Lys-4-aminopiperidine-4-carboxamide-OH |
| Compound 105 | D-Phe-D-Phe(4-F)-D-Leu-D-Lys-4-aminopiperidine-4-carboxamide-Lys-NH₂ |
| Compound 106 | D-Phe-D-Phe(4-F)-D-Leu-D-Lys-4-aminopiperidine-4-carboxamide-Lys-OH |
| Compound 107 | D-Phe-D-Phe(4-F)-D-Leu-D-Lys-4-aminopiperidine-4-carboxamide-D-Lys-NH₂ |
| Compound 108 | D-Phe-D-Phe(4-F)-D-Leu-D-Lys-4-aminopiperidine-4-carboxamide-D-Lys-OH |
| Compound 109 | D-Phe-D-Phe(4-Cl)-D-Leu-D-Lys-4-aminopiperidine-4-carboxamide -NH₂ |
| Compound 110 | D-Phe-D-Phe(4-Cl)-D-Leu-D-Lys-4-aminopiperidine-4-carboxamide -OH |
| Compound 111 | D-Phe-D-Phe(4-Cl)-D-Leu-D-Lys-4-aminopiperidine-4-carboxamide -Lys-NH₂ |
| Compound 112 | D-Phe-D-Phe(4-Cl)-D-Leu-D-Lys-4-aminopiperidine-4-carboxamide -Lys-OH |
| Compound 113 | D-Phe-D-Phe(4-Cl)-D-Leu-D-Lys-4-aminopiperidine-4-carboxamide -D-Lys-NH₂ |
| Compound 114 | D-Phe-D-Phe(4-Cl)-D-Leu-D-Lys-4-aminopiperidine-4-carboxamide -D-Lys-OH |
| Compound 115 | D-Phe-D-Phe(4-Br)-D-Leu-D-Lys-4-aminopiperidine-4-carboxamide -NH₂ |
| Compound 116 | D-Phe-D-Phe(4-Br)-D-Leu-D-Lys-4-aminopiperidine-4-carboxamide -OH |
| Compound 117 | D-Phe-D-Phe(4-Br)-D-Leu-D-Lys-4-aminopiperidine-4-carboxamide -Lys-NH₂ |
| Compound 118 | D-Phe-D-Phe(4-Br)-D-Leu-D-Lys-4-aminopiperidine-4-carboxamide -Lys-OH |
| Compound 119 | D-Phe-D-Phe(4-Br)-D-Leu-D-Lys-4-aminopiperidine-4-carboxamide -D-Lys-NH₂ |
| Compound 120 | D-Phe-D-Phe(4-Br)-D-Leu-D-Lys-4-aminopiperidine-4-carboxamide -D-Lys-OH |
| Compound 121 | D-Phe-D-Phe(4-I)-D-Leu-D-Lys-4-aminopiperidine-4-carboxamide-NH₂ |
| Compound 122 | D-Phe-D-Phe(4-I)-D-Leu-D-Lys-4-aminopiperidine-4-carboxamide-OH |
| Compound 123 | D-Phe-D-Phe(4-I)-D-Leu-D-Lys-4-aminopiperidine-4-carboxamide-Lys-NH₂ |
| Compound 124 | D-Phe-D-Phe(4-I)-D-Leu-D-Lys-4-aminopiperidine-4-carboxamide-Lys-OH |
| Compound 125 | D-Phe-D-Phe(4-I)-D-Leu-D-Lys-4-aminopiperidine-4-carboxamide-D-Lys-NH₂ |
| Compound 126 | D-Phe-D-Phe(4-I)-D-Leu-D-Lys-4-aminopiperidine-4-carboxamide-D-Lys-OH |
| Compound 127 | D-Phe-D-Phe(4-Me)-D-Leu-D-Lys-4-aminopiperidine-4-carboxamid e-Lys-NH₂ |
| Compound 128 | D-Phe-D-Phe(4-Me)-D-Leu-D-Lys-4-aminopiperidine-4-carboxamid e-Lys-OH |
| Compound 129 | D-Phe-D-Phe(4-Me)-D-Leu-D-Lys-4-aminopiperidine-4-carboxamid e-D-Lys-NH₂ |
| Compound 130 | D-Phe-D-Phe(4-Me)-D-Leu-D-Lys-4-aminopiperidine-4-carboxamid e-D-Lys-OH |
| Compound 131 | D-Phe-D-Phe(4-Et)-D-Leu-D-Lys-4-aminopiperidine-4-carboxamide-Lys-NH₂ |
| Compound 132 | D-Phe-D-Phe(4-Et)-D-Leu-D-Lys-4-aminopiperidine-4-carboxamide-Lys-OH |
| Compound 133 | D-Phe-D-Phe(4-Et)-D-Leu-D-Lys-4-aminopiperidine-4-carboxamide-D-Lys-NH₂ |
| Compound 134 | D-Phe-D-Phe(4-Et)-D-Leu-D-Lys-4-aminopiperidine-4-carboxamide-D-Lys-OH |
| Compound 135 | D-Phe-D-Phe(4-Ipr)-D-Leu-D-Lys-4-aminopiperidine-4-carboxamide -Lys-NH₂ |
| Compound 136 | D-Phe-D-Phe(4-Ipr)-D-Leu-D-Lys-4-aminopiperidine-4-carboxamide -Lys-OH |
| Compound 137 | D-Phe-D-Phe(4-Ipr)-D-Leu-D-Lys-4-aminopiperidine-4-carboxamide -D-Lys-NH₂ |
| Compound 138 | D-Phe-D-Phe(4-Ipr)-D-Leu-D-Lys-4-aminopiperidine-4-carboxamide -D-Lys-OH |
| Compound 139 | D-Phe-D-Phe(4-NH₂)-D-Leu-D-Lys-4-aminopiperidine-4-carboxami de-Lys-NH₂ |
| Compound 140 | D-Phe-D-Phe(4-NH₂)-D-Leu-D-Lys-4-aminopiperidine-4-carboxami de-Lys-OH |
| Compound 141 | D-Phe-D-Phe(4-NH₂)-D-Leu-D-Lys-4-aminopiperidine-4-carboxami de-D-Lys-NH₂ |
| Compound 142 | D-Phe-D-Phe(4-NH₂)-D-Leu-D-Lys-4-aminopiperidine-4-carboxami de-D-Lys-OH |

### Example 2: determination of activities of κ receptor agonists

### 1. Determination Method

Gi-coupled human **κ** opioid receptor (OPRK1), upon stimulation with its specific opioid receptor agonists, can inhibit the intracellular adenylate cyclase pathway, and decrease cAMP levels. Forskolin can stimulate cAMP release in cell lines highly expressing opioid receptors. By stimulating cell lines stably transfected with opioid receptors, the inhibitory effect of a test compound on forskolin-stimulated cAMP release can be measured to determine the activity of the compound. The relative light units (RLU) from the cells after stimulation with different doses were measured via the homogeneous time-resolved fluorescence technique (HTRF), and the EC₅₀ value of the agonist was subsequently calculated. This activity determination method is a currently commonly used method for determining the activities of opioid receptor agonists at home and abroad.

A CHO-K1 cell line stably expressing opioid receptors was used. Stably transfected cells were stimulated with various concentrations of agonists (under the stimulation of a certain concentration of forskolin). The relative light units from the cells after stimulation with different doses were measured, from which the EC₅₀ value of the agonist was subsequently obtained by calculation.

### 2. Results of determination

The results of the determination are presented in the table below:

**Table 3**

| **Compound Number** | **EC₅₀ (pmol)** | **Relative Activity%** |
|---|---|---|
| Difelikefalin | 607.2 | 100.0 |
| Compound 1 | 115.7 | 524.8 |
| Compound 2 | 117.0 | 519.0 |
| Compound 3 | 39.4 | 1541.1 |
| Compound 4 | 39.9 | 1521.8 |
| Compound 5 | 41.0 | 1481.0 |
| Compound 6 | 40.2 | 1510.4 |
| Compound 7 | 42.2 | 1438.9 |
| Compound 8 | 41.7 | 1456.1 |
| Compound 9 | 47.7 | 1273.0 |
| Compound 10 | 46.3 | 1311.4 |
| Compound 11 | 53.4 | 1137.1 |
| Compound 12 | 54.1 | 1122.4 |
| Compound 13 | 66.9 | 907.6 |
| Compound 14 | 65.5 | 927.0 |
| Compound 15 | 42.9 | 1415.4 |
| Compound 16 | 43.1 | 1408.8 |
| Compound 17 | 45.3 | 1340.4 |
| Compound 18 | 46.0 | 1320.0 |
| Compound 19 | 47.9 | 1267.6 |
| Compound 20 | 48.2 | 1259.8 |
| Compound 21 | 50.1 | 1212.0 |
| Compound 22 | 50.6 | 1200.0 |
| Compound 23 | 55.4 | 1096.0 |
| Compound 24 | 56.1 | 1082.4 |
| Compound 25 | 68.4 | 887.7 |
| Compound 26 | 67.1 | 904.9 |
| Compound 27 | 46.2 | 1314.3 |
| Compound 28 | 45.0 | 1349.3 |
| Compound 29 | 45.6 | 1331.6 |
| Compound 30 | 46.5 | 1305.8 |
| Compound 31 | 53.2 | 1141.4 |
| Compound 32 | 54.1 | 1122.4 |
| Compound 33 | 53.6 | 1132.8 |
| Compound 34 | 53.0 | 1145.7 |
| Compound 35 | 54.9 | 1106.0 |
| Compound 36 | 55.2 | 1100.0 |
| Compound 37 | 56.1 | 1082.4 |
| Compound 38 | 55.4 | 1096.0 |
| Compound 39 | 60.2 | 1008.6 |
| Compound 40 | 58.0 | 1046.9 |
| Compound 41 | 59.6 | 1018.8 |
| Compound 42 | 58.5 | 1037.9 |
| Compound 43 | 88.3 | 687.7 |
| Compound 44 | 86.7 | 700.3 |
| Compound 45 | 85.9 | 706.9 |
| Compound 46 | 87.0 | 697.9 |
| Compound 47 | 43.9 | 1383.1 |
| Compound 48 | 43.2 | 1405.6 |
| Compound 49 | 44.5 | 1364.5 |
| Compound 50 | 44.9 | 1352.3 |
| Compound 51 | 47.7 | 1273.0 |
| Compound 52 | 48.5 | 1252.0 |
| Compound 53 | 46.3 | 1311.4 |
| Compound 54 | 47.1 | 1289.2 |
| Compound 55 | 52.1 | 1165.5 |
| Compound 56 | 50.6 | 1200.0 |
| Compound 57 | 51.4 | 1181.3 |
| Compound 58 | 51.5 | 1179.0 |
| Compound 59 | 69.4 | 874.9 |
| Compound 60 | 71.3 | 851.6 |
| Compound 61 | 70.6 | 860.1 |
| Compound 62 | 70.1 | 866.2 |
| Compound 63 | 90.5 | 670.9 |
| Compound 64 | 89.9 | 675.4 |
| Compound 65 | 88.4 | 686.9 |
| Compound 66 | 89.5 | 678.4 |
| Compound 67 | 127.1 | 477.7 |
| Compound 68 | 125.6 | 483.4 |
| Compound 69 | 42.4 | 1432.1 |
| Compound 70 | 43.7 | 1389.5 |
| Compound 71 | 41.0 | 1481.0 |
| Compound 72 | 42.6 | 1425.4 |
| Compound 73 | 46.8 | 1297.4 |
| Compound 74 | 45.7 | 1328.7 |
| Compound 75 | 47.3 | 1283.7 |
| Compound 76 | 46.6 | 1303.0 |
| Compound 77 | 55.1 | 1102.0 |
| Compound 78 | 53.2 | 1141.4 |
| Compound 79 | 54.9 | 1106.0 |
| Compound 80 | 54.5 | 1114.1 |
| Compound 81 | 56.2 | 1080.4 |
| Compound 82 | 56.9 | 1067.1 |
| Compound 83 | 55.7 | 1090.1 |
| Compound 84 | 56.1 | 1082.4 |
| Compound 85 | 125.5 | 483.8 |
| Compound 86 | 121.2 | 501.0 |
| Compound 87 | 42.2 | 1438.9 |
| Compound 88 | 43.5 | 1395.9 |
| Compound 89 | 43.6 | 1392.7 |
| Compound 90 | 44.2 | 1373.8 |
| Compound 91 | 110.4 | 550.0 |
| Compound 92 | 108.7 | 558.6 |
| Compound 93 | 51.2 | 1185.9 |
| Compound 94 | 52.6 | 1154.4 |
| Compound 95 | 50.5 | 1202.4 |
| Compound 96 | 49.4 | 1229.1 |
| Compound 97 | 147.6 | 411.4 |
| Compound 98 | 145.2 | 418.2 |
| Compound 99 | 72.6 | 836.4 |
| Compound 100 | 71.6 | 848.0 |
| Compound 101 | 72.8 | 834.1 |
| Compound 102 | 73.0 | 831.8 |
| Compound 103 | 220.8 | 275.0 |
| Compound 104 | 215.4 | 281.9 |
| Compound 105 | 79.6 | 762.8 |
| Compound 106 | 78.0 | 778.5 |
| Compound 107 | 77.3 | 785.5 |
| Compound 108 | 78.5 | 773.5 |
| Compound 109 | 151.0 | 402.1 |
| Compound 110 | 148.6 | 408.6 |
| Compound 111 | 52.1 | 1165.5 |
| Compound 112 | 50.8 | 1195.3 |
| Compound 113 | 49.4 | 1229.1 |
| Compound 114 | 51.5 | 1179.0 |
| Compound 115 | 137.4 | 441.9 |
| Compound 116 | 135.5 | 448.1 |
| Compound 117 | 65.0 | 934.2 |
| Compound 118 | 63.8 | 951.7 |
| Compound 119 | 61.5 | 987.3 |
| Compound 120 | 62.7 | 968.4 |
| Compound 121 | 122.4 | 496.1 |
| Compound 122 | 120.1 | 505.6 |
| Compound 123 | 53.5 | 1135.0 |
| Compound 124 | 55.8 | 1088.2 |
| Compound 125 | 55.0 | 1104.0 |
| Compound 126 | 56.7 | 1070.9 |
| Compound 127 | 157.4 | 385.8 |
| Compound 128 | 159.7 | 380.2 |
| Compound 129 | 151.5 | 400.8 |
| Compound 130 | 152.6 | 397.9 |
| Compound 131 | 133.6 | 454.5 |
| Compound 132 | 130.4 | 465.6 |
| Compound 133 | 131.5 | 461.7 |
| Compound 134 | 132.7 | 457.6 |
| Compound 135 | 146.3 | 415.0 |
| Compound 136 | 149.2 | 407.0 |
| Compound 137 | 143.5 | 423.1 |
| Compound 138 | 145.9 | 416.2 |
| Compound 139 | 112.4 | 540.2 |
| Compound 140 | 115.0 | 528.0 |
| Compound 141 | 111.8 | 543.1 |
| Compound 142 | 109.7 | 553.5 |

The results of the aforementioned experiments show that the compounds provided in the present disclosure have an activity much greater than that of Difelikefalin. Compounds 1 to 142 provided in the Examples have an activity 3 to 15 times that of Difelikefalin.

Finally, it should be noted that the foregoing embodiments are provided for the purpose of illustrating the technical solutions of the present disclosure, and are not intended to be limiting. Although the present disclosure has been described in detail with reference to the aforementioned embodiments, a person of ordinary skill in the art should understand that modifications to the technical solutions described, or equivalent substitutions for some or all of the technical features thereof, may still be made. Such modifications or substitutions, however, do not cause the essence of the respective technical solutions to depart from the scope of the technical solutions set forth in the embodiments of the present disclosure.

## Claims

1. A κ-opioid receptor agonist as represented by formula I, a stereisomer thereof, and a pharmaceutically acceptable salt thereof:
D-Phe-AA1-AA2-D-Lys-4-aminopiperidine-4-carboxamide-AA3-AA4-AA5 (formula I)
wherein, AA1 is modified or unmodified D-Phe, or modified or unmodified D-Tyr;
AA2 is D-Leu, or D-cyclopropylalanine;
AA3 is -(PEGₘ₁(CH₂)ₘ₂CO)ₘ₃-, or (AA6)ₘ₄, or is absent, wherein:
m₁ is an integer ranging from 1 to 10;
m₂ is an integer ranging from 1 to 5;
m₃ is an integer ranging from 1 to 5;
m₄ is an integer ranging from 1 to 5;
AA6 is AEEA, or Gly, or D-Ala, or L-Ala, or D-Leu, or L-Leu, or D-Phe, or L-Phe, or D-Ser, or L-Ser, or D-Thr, or L-Thr, or D-Tyr, or L-Tyr, or D-Asp, or L-Asp, or D-Glu, or L-Glu, or D-Gln, or L-Gln, or D-Lys, or L-Lys, or D-Arg, or L-Arg, or D-His, or L-His;
AA4 is (AA7)ₙ, or is absent, wherein:
n is an integer ranging from 1 to 10;
AA7 is D-Lys, or L-Lys, or D-Dap, or L-Dap, or D-Dab, or L-Dab, or D-Orn, or L-Orn, or D-Dah, or L-Dah, or D-Dao, or L-Dao;
AA5 is OH, or NH₂.

2. The κ-opioid receptor agonist, the stereisomer thereof, and the pharmaceutically acceptable salt thereof according to claim 1, wherein AA1 is modified D-Phe, or modified D-Tyr; the modified D-Phe is selected from the group consisting of D-Phe(2-F), D-Phe(4-F), D-Phe(2-Cl), D-Phe(4-Cl), D-Phe(2-Br), D-Phe(4-Br), D-Phe(2-I), D-Phe(4-I), D-Phe(4-Me), D-Phe(4-Et), D-Phe(4-Ipr), D-Phe(4-NH₂), D-Phe(4-NHCH₃), D-Phe(4-NHCH₂CH₃), D-Phe(4-NHCH(CH₃)₂), D-Phe(4-N(CH₃)₂), and D-Phe(4-N(CH₃) CH₂CH₃); and the modified D-Tyr is selected from the group consisting of D-Tyr(Me), D-Tyr(Et), and D-Tyr(Ipr).

3. The κ-opioid receptor agonist, the stereisomer thereof, and the pharmaceutically acceptable salt thereof according to claim 2, wherein the κ-opioid receptor agonist has a structure as represented by formula II:
D-Phe-AA1-AA2-D-Lys-4-aminopiperidine-4-carboxamide-R-(AA8)ₚ-NH_{Z}(OH) (formula II)
wherein, AA1 is modified D-Phe, or modified D-Tyr;
AA2 is D-Leu, or D-cyclopropylalanine;
R is present or absent, and when R is present, R is selected from the group consisting of PEGₘ₁CH₂CO-,-AEEAₙ₁-, and-Glyₙ₂-; wherein:
m₁ is an integer ranging from 1 to 5, and specifically, is selected from the group consisting of 1, 2, 3, 4, and 5;
n₁ is an integer ranging from 1 to 5, and specifically, is selected from the group consisting of 1, 2, 3, 4, and 5;
n₂ is an integer ranging from 1 to 5, and specifically, is selected from the group consisting of 1, 2, 3, 4, and 5;
AA8 is Lys, or D-Lys; p is an integer ranging from 0 to 6, and specifically, is selected from the group consisting of 0, 1, 2, 3, 4, 5, and 6; when p is 0, AA8 is absent.

4. The κ-opioid receptor agonist, the stereisomer thereof, and the pharmaceutically acceptable salt thereof according to claim 3, wherein the κ-opioid receptor agonist has a structure as represented by formula III:
D-Phe-D-Tyr(X)-D-Leu-D-Lys-4-aminopiperidine-4-carboxamide-R₁-(AA8)ₚ₁-NH₂(OH) (formula III)
wherein, D-tyr(X) represents modified D-Tyr; the modified D-Tyr is selected from the group consisting of D-Tyr(Me), D-Tyr(Et), D-Tyr(CH₂CF₃), and D-Tyr(Ipr);
R₁ is present or is absent; when R₁ is present, R₁ is selected from the group consisting of-PEGₖ₁CH₂CO-, and-Glyₙ₂-; wherein:
k₁ is an integer ranging from 1 to 5, and specifically, is selected from the group consisting of 1, 2, 3, 4, and 5;
n₂ is an integer ranging from 1 to 5, and specifically, is selected from the group consisting of 1, 2, 3, 4, and 5;
AA8 is Lys, or D-Lys; p₁ is an integer ranging from 0 to 6, and specifically, is selected from the group consisting of 0, 1, 2, 3, 4, 5, and 6; when p₂ is 0, AA8 is absent.

5. The κ-opioid receptor agonist, the stereisomer thereof, and the pharmaceutically acceptable salt thereof according to claim 3, wherein the κ-opioid receptor agonist has a structure as represented by formula IV:
D-Phe-D-Tyr(X)-D-cyclopropylalanine-D-Lys-4-aminopiperidine-4-carboxamide -R₂-(AA8)ₚ₂-NH₂(OH) (formula IV)
wherein, D-tyr(X) represents modified D-Tyr; the modified D-Tyr is selected from the group consisting of D-Tyr(Me), D-Tyr(Et), and D-Tyr(Ipr);
R₂ is present or is absent; when R₂ is present, R₂ is-PEGₖ₂CH₂CO-; wherein:
k₂ is an integer ranging from 1 to 5, and specifically, is selected from the group consisting of 1, 2, 3, 4, and 5;
in most preferred embodiments of the present disclosure, the k₂ is 1, 2, or 3;
AA8 is Lys, or D-Lys; p₃ is an integer ranging from 0 to 6, and specifically, is selected from the group consisting of 0, 1, 2, 3, 4, 5, and 6; when p₃ is 0, AA8 is absent.

6. The κ-opioid receptor agonist, the stereisomer thereof, and the pharmaceutically acceptable salt thereof according to claim 3, wherein the κ-opioid receptor agonist has a structure as represented by formula V:
D-Phe-D-Phe(X)-D-Leu-D-Lys-4-aminopiperidine-4-carboxamide-(AA8)ₚ₃-NH₂(OH) (formula V)
wherein, D-Phe(X) represents modified D-Phe; the modified D-Phe is selected from the group consisting of D-Phe(2-F), D-Phe(4-F), D-Phe(2-Cl), D-Phe(4-Cl), D-Phe(2-Br), D-Phe(4-Br), D-Phe(2-I), D-Phe(4-I), D-Phe(4-Me), D-Phe(4-Et), D-Phe(4-Ipr), D-Phe(4-NH₂);
AA8 is Lys, or D-Lys; p₃ is an integer ranging from 0 to 6, and specifically, is selected from the group consisting of 0, 1, 2, 3, 4, 5, and 6; when p₃ is 0, AA8 is absent.

7. The κ-opioid receptor agonist, the stereisomer thereof, and the pharmaceutically acceptable salt thereof according to any one of the claims 1 to 6, wherein the κ-opioid receptor agonist has one or more structural formulas selected from the group consisting of 1) to 10):
1) D-Phe-D-Tyr(X)-D-Leu-D-Lys-4-aminopiperidine-4-carboxylic acid-NH₂(OH)
2) D-Phe-D-Tyr(X)-D-Leu-D-Lys-4-aminopiperidine-4-carboxamide-NH₂(OH)
3) D-Phe-D-Tyr(X)-D-Leu-D-Lys-4-aminopiperidine-4-carboxamide-(AA8)_{q}-NH₂(OH)
4) D-Phe-D-Tyr(X)-D-Leu-D-Lys-4-aminopiperidine-4-carboxamide-PEGₖ₁CH₂CO-AA8-NH₂(OH)
5) D-Phe-D-Tyr(X)-D-Leu-D-Lys-4-aminopiperidine-4-carboxamide-Glyₙ₂-AA8-NH₂(OH)
6) D-Phe-D-Tyr(X)-D-cyclopropylalanine-D-Lys-4-aminopiperidine-4-carboxamide-NH₂ (OH)
7) D-Phe-D-Tyr(X)-D-cyclopropylalanine-D-Lys-4-aminopiperidine-4-carboxamide-AA8-NH₂(OH)
8) D-Phe-D-Tyr(X)-D-cyclopropylalanine-D-Lys-4-aminopiperidine-4-carboxamide-PEGₖ₂ CH₂CO-AA8-NH₂(OH)
9) D-Phe-D-Phe(X)-D-Leu-D-Lys-4-aminopiperidine-4-carboxamide-NH₂(OH)
10) D-Phe-D-Phe(X)-D-Leu-D-Lys-4-aminopiperidine-4-carboxamide-AA8-NH₂(OH),
in aforementioned structural formulas, AA8 is Lys, or D-Lys; wherein:
in formula 1), D-Tyr(X) is D-Tyr(Et);
in formulas 2) to 3), D-Tyr(X) is D-Tyr(Me), or D-Tyr(Et), or D-Tyr(CH₂CF₃), or D -Tyr(Ipr), and q is 1, or 2, or 3, or 4, or 5, or 6;
in formulas 4) to 8), D-Tyr(X) is D-Tyr(Et),
k₁ is 1, or 2, or 3, or 4, or 5;
n₂ is 1, or 2, or 3, or 4, or 5;
k₂ is 1, or 2, or 3;
in formula 9), D-Phe(X) is D-Phe(4-F), or D-Phe(4-Cl), or D-Phe(4-Br), or D-Phe(4-I);
in formula 10), D-Phe(X) is D-Phe(4-F), or D-Phe(4-Cl), or D-Phe(4-Br), or D-Phe(4 -I), or D-Phe(4-Me), or D-Phe(4-Et), or D-Phe(4-Ipr), or D-Phe(4-NH₂).

8. A pharmaceutical composition, comprising the κ-opioid receptor agonist, the stereisomer thereof, and the pharmaceutically acceptable salt thereof according to any one of claims 1 to 7.

9. The pharmaceutical composition according to claim 8, wherein the pharmaceutical composition further comprises a pharmaceutically acceptable carrier.

10. Use of the x-opioid receptor agonist, the stereisomer thereof, and the pharmaceutically acceptable salt thereof according to any one of claims 1 to 7, and/or the pharmaceutical composition according to any one of claims 8 to 9, in the manufacture of a medicament for preventing and/or relieving pruritus and/or an analgesic.
